# EUROPEAN PATENT SPECIFICATION

(11) **EP 1 483 015 B1**
(45) Date of publication and mention of the grant of the patent: **26.08.2009**
(21) Application number: 03708339.1
(22) Date of filing: 10.03.2003
(51) Int. Cl.: A61N 1/00, A61N 1/39

(54) **MEDICAL DEVICE**
MEDIZINISCHE VORRICHTUNG
DISPOSITIF MEDICAL

(30) Priority: 09.03.2002 GB 0205567
(43) Date of publication of application: 08.12.2004
(73) Proprietor: Ixa Medical Products LLP, Cheltenham, Gloucestershire GL50 1SS (GB)
(72) Inventor: MILLS, Desmond Bryan, IXA Medical Products LLP, Cheltenham, Gloucestershire GL50 1YD (GB); HERBERT, Kevin, IXA Medical Products LLP, Cheltenham, Gloucestershire GL50 1YD (GB)
(74) Representative: Newell, William Joseph
(86) International application number: PCT/GB2003/001009
(87) International publication number: WO 2003/076005

(56) References cited:
- EP-B- 0 813 840
- FR-A- 2 746 508
- US-A- 4 141 351
- US-A- 4 576 170
- US-A- 5 864 619
- US-A- 5 879 374

## Description

The present invention relates to a medical device and in particular to a cardiopulmonary resuscitation device, such as an automated external defibrillator (AED), that is activated by whether or not electrodes, for administering treatment, are associated with the device.

In adults, the commonest primary arrhythmias at the onset of cardiac arrest are ventricular fibrillation (VF), or pulseless ventricular tachycardia (VT). When such arrhythmias occur, the primary form of therapy is defibrillation, which should be carried out with the minimum of delay in order maximise the patient's chances of survival. If treatment is delayed, the chances of success of reviving a patient with an otherwise relatively treatable arrhythmia such as VF, declines rapidly with the passage of time. If an electrocardiogram is taken of the patients experiencing, for example VF, the amplitude and waveform of a patient's electrocardiogram indicates that changes occur in the patient's body such as the depletion of myocardial energy stores and lack of oxygenation. Basic life support treatment will not reverse these changes in the patient and can only slow the rate of deterioration. It is a major priority to minimise risk of this deterioration occurring by ensuring that there is the minimum delay between the onset of a cardiac arrest and the administration of treatment by defibrillation using an AED in order to provide a shock to regulate the patient's heart rhythm. Defibrillation may be possible for several minutes after a cardiac arrest occurs but the chances of survival are optimised if treatment is given in the period of around 90 seconds after the onset of cardiac arrest.

In general, administration of shocks to patients is only given by qualified personnel, such as paramedics specifically trained to use AEDs. The training ensures that personnel use such equipment in a way that there is minimal risk off an inappropriate shock being given to a patient. For example, if a patient is not experiencing arrhythmia, and a shock is administered, then the heart rate of an otherwise healthy patient could be disturbed and a cardiac arrest triggered.

As presently only trained personnel are able to use AED's, this results in a delay in treating a patient as they have to reach the patient. If an ambulance has to be called, this delay can often be several minutes by which time a patient may have died. A device according to the preamble of claim 1 is known from US 4 653 474.

Consequently, there is a need for providing a medical device such as an AED that is simple to use and which can be used by persons at the scene of an incident who may not necessarily be qualified in administering treatment to an individual.

According to the invention there is provided a medical device having a control circuit adapted to operate one or more electrodes in response to analysis of a patient's condition by the medical device, **characterised in that** the medical device includes a support for an electrode, whereby when the electrode is associated with the support, the control circuit is deactivated but on removal of the electrode from the support, the control circuit is activated.

Preferably, there are one or more supports each being associated with an electrode.

It is envisaged that the support or supports comprise apertures in a housing of the medical device. Preferably, the electrodes may be inserted in the apertures thereby interrupting the control circuit either completely or to a substantial extent. When the circuit is interrupted to a substantial extent, there may be a trickle charge through the electrode so that the medical device remains on standby.

Preferably, when the device is on standby, the condition of the electrode may be monitored by the charge passing through it. This may be by measuring electrical impedance or resistance across the electrode.

It is preferred that the electrode is contained within a pouch. The pouch protects the electrode prior to use.

It is envisaged that the medical device includes an electro-detection circuit for detecting the presence of an electrode or an electrical pouch, which is inserted in the housing. It is envisaged that the circuit may comprise a switch device that is activated when the pouch is inserted or removed from the housing by displacement of a mechanical element. The switch may be in the form of a spring connector in a preferred arrangement. Alternatively, the detection circuit may use a field effect sensor to detect the presence of a pouch or electrode between the emitter and sensor. Also, the detection circuit may include a light source and detector, with the light source being interrupted when an electrode or pouch is inserted in the housing. When the electrode/pouch is removed from the housing, the detector senses light emission and then acts as a switch to activate the medical device. It is envisaged that a combination of such switching elements may be used so that a back up mechanism can be provided for detection and activation of the device.

It is preferred that when the electrode or pouch is removed from the detection circuit, the medical device is switched on ready to deliver treatment, such as a shock via the electrode. In a further embodiment, when the electrode is removed, rather than the device being immediately activated ready for treatment, a self testing diagnostic routine is activated so that self checks can be carried out by the device to ensure that it is functioning correctly prior to use on the patient. It is envisaged that the operation of the removal of the pouch or electrode may directly operate the device or may cause indirect operation of the device by triggering a further prompt mechanism in the device for starting either the self-diagnostic routine or treatment of the patient.

In a further embodiment, activation of a switch allows, either directly or indirectly, a power line from a battery system in the device to the control system to become activated. This causes a processor or sub-processor to change its status from idle to active. The device can then be activated by switching on, or alternatively a self-test routine may be activated depending on the preset instructions of the device. Whether a self-test occurs or not can be selected by the medical device operator/installer and it may be the case that self-testing is not carried out where there is or is likely to be an ultra emergency, for example the patient is on the point of dying.

In a further embodiment, a processor or sub-processor may actively or passively monitor the status of the switching mechanism. A change in status would then alert the processor or sub-processor and would cause power to be activated for the medical device or a self-test may be activated depending again on what the prior programmed instructions are for the device.

The medical device may be defined as a device that delivers or monitors the condition of a patient by using electrodes. In particular, the invention is concerned with cardiopulmonary resuscitation devices such as defibrillators or heart monitors, but also the invention is also applicable to other types of devices using electrodes placed on a patient's body such as electrocardigrams (ECGs) or electroencephalogram EEGs.

An embodiment of the invention will now be described, by way of example only, with reference to the following figures in which:
Figure 1 is a schematic diagram of the circuitry associated with the device according to the invention;
Figure 2 shows an electrode in-situ device within a detection circuit; and
Figure 3 shows a schematic diagram of the circuitry incorporating an electrode.

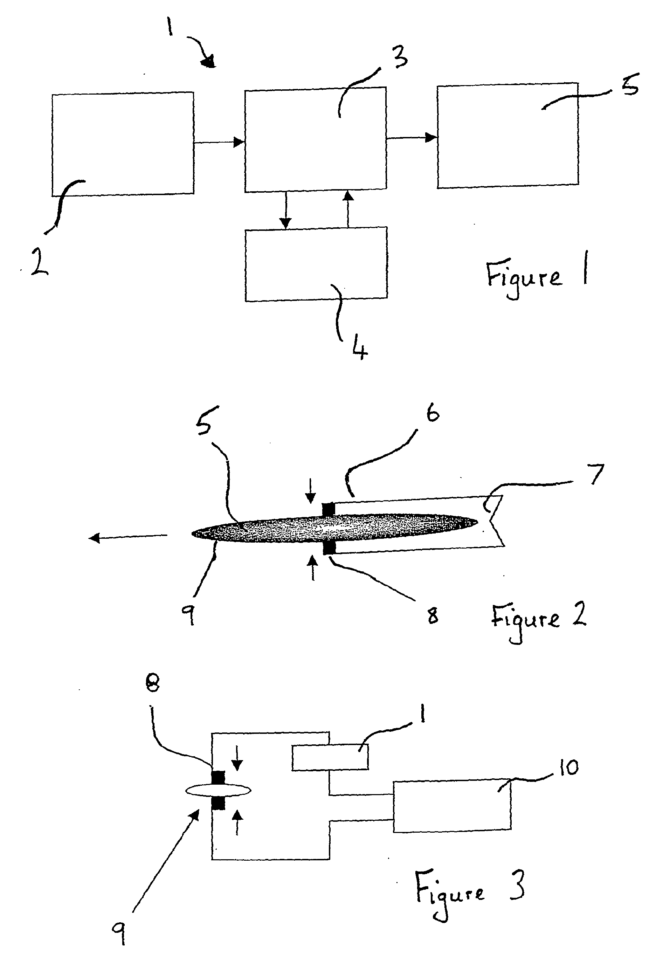

As shown in Figure 1, a circuit for operating a medical device, which in this case is a defibrillator, is generally shown as 1. The circuit comprises a power source 2 and a switching circuit 3, which switches charge between the power source 2 and capacitors 4 so that charge can be delivered to electrodes 5 which are placed on the patient's chest during treatment. The defibrillator has a housing (not shown) with an aperture 6 in the form of a slot in the housing. An electrode 5, which is contained in this case in packaging 9 in the form of a pouch around the electrode, is inserted in the aperture 6. On either side of the opening of the aperture 6 are electrical connectors 8. These may be in the form of metal contacts. The connectors 8 are held apart by spring connectors 7 at the end of the aperture opposed to the opening. Insertion of the electrode 5 in the aperture 6 forces apart the connectors 8. The spring connectors are held in tension by this forcing apart. When the electrode is removed from the aperture in the direction of the arrow shown, the tension in the spring connectors is released and the electric connectors 8 come together to meet so completing the circuit of the defibrillator. When the circuit is completed, the defibrillator becomes activated by flow of charge from the battery 2. At this point, the defibrillator may be ready for activation or alternatively, depending upon how the defibrillator is set up or programmed, a self-test mechanism may occur.

Figure 3 shows a schematic diagram of an electrode inserted within the circuitry of the defibrillator. A power source, including the battery 2 which in turn is in communication with a processor 10 which can sense the presence of electrode 5 or electrode packaging 9. A processor 10 can monitor the condition of the electrode 5. Alternatively, or in addition to monitoring the electrode condition, once the electrode is removed from the defibrillator, it can act activate circuitry to switch the defibrillator on or it can start a pre-check routine to ensure that the defibrillator is in a condition whereby it may deliver charge effectively to the patient.

It is to be understood that the figures refer to a preferred embodiment of the invention. It is may be that there are multiple apertures, each for housing a separate electrode or alternatively, a single aperture may house a number of electrode, with the medical device only being activated when the first or final or any electrode is removed. This arrangement would be particularly useful where pairs of electrodes are used to treat a patient.

Further electrodes need not necessarily be held in protective pouches, it may be that the aperture has integral protective means such as cushioning for protecting the electrode.

Having described specific embodiments of the invention, other embodiments and uses will become apparent to those skilled in the art without departing from the inventive concepts described.

## Claims

1. A medical device having a control circuit adapted to operate one or more electrodes in response to analysis of a patient's condition by the medical device, whereby the medical device includes a support for an electrode, **characterised in that** the device is configured such that when the electrode is associated with the support, the control circuit is deactivated but on removal of the electrode from the support, the control circuit is activated.

2. A medical device according to Claim 1, wherein the medical device has one or more supports, each being arranged to be associated with a respective electrode.

3. A medical device according to Claim 1 or Claim 2, wherein the support or supports comprise apertures in a housing of the medical device configured such that when the or each electrode is inserted in respective apertures, the control circuit is interrupted either completely or to a substantial extent by the presence of the electrode(s) in the or each aperture in the housing.

4. A medical device according to Claim 3, wherein when the control circuit is interrupted, said control circuit is arranged to allow the flow of a trickle charge through the or each electrode so that the medical device can remain on standby.

5. A medical device according to Claim 4, including a monitor, said medical device being configured such that when the medical device is on standby, the monitor is arranged to monitor the condition of the or each electrode as a function of the charge passing through it.

6. A medical device according to Claim 5, wherein the monitor is arranged to measure electrical impedance or resistance across the electrode.

7. A medical device according to any preceding claim, wherein the medical device includes a detection circuit associated with the or each aperture in said housing, the detection circuit being arranged to detect the presence of an electrode or a pouch containing an electrode when inserted in the aperture(s) in the housing.

8. A medical device according to Claim 7, including a switch for switching on power to the control circuit so that the medical device is activatable on removal of the electrode/pouch to allow power

9. A medical device according to claim 8, wherein the detection circuit includes a light source and detector, with the detector being arranged to detect interruption of the light source when an electrode/pouch is inserted in the housing and light emission when the electrode/pouch is removed from the housing, the detector then acting as the switch to activate the medical device.

10. A medical device according to Claim 9, wherein the medical device includes a combination of switching elements to detect and activate said medical device.

11. A medical device according to any preceding claim, including a self test mechanism configured such that when electrode(s) are removed from the housing, a circuit is completed in the medical device which can either cause the medical device to be activated ready for use or can activate the self-testing mechanism so that checks can be carried out to ensure said medical device is functioning correctly prior to it being used on the patient.

12. A medical device according to Claim 11, wherein the self-testing mechanism is arranged to actively or passively monitor switching in the medical device as a result of removal or insertion of electrodes in the housing, a change in status of the switching then alerting a processor or sub-processor so that the control circuit can be activated.

13. A medical device according to any preceding claim, which is a cardio-pulmonary resuscitation device.

## Patentansprüche

1. Medizinische Vorrichtung mit einer Steuerschaltung, die eine oder mehrere Elektroden in Abhängigkeit von der Analyse eines Patientenzustandes betreiben kann, wobei die medizinische Vorrichtung mit einem Träger für eine Elektrode versehen ist, **dadurch gekennzeichnet, daß** die Vorrichtung so ausgebildet ist, daß dann, wenn die Elektrode mit dem Träger verbunden wird, die Steuerschaltung ausgeschaltet wird, jedoch dann, wenn die Elektrode von dem Träger entfernt wird, die Steuerschaltung aktiviert wird.

2. Medizinische Vorrichtung nach Anspruch 1, **dadurch gekennzeichnet, daß** die medizinische Vorrichtung einen oder mehrere Träger aufweist, von denen jeder so angeordnet ist, daß er mit einer entsprechenden Elektrode verbunden ist.

3. Medizinische Vorrichtung nach Anspruch 1 oder 2, **dadurch gekennzeichnet, daß** der oder die Träger in einem Gehäuse der medizinischen Vorrichtung Öffnungen aufweisen, die so ausgebildet sind, daß dann, wenn die oder jede Elektrode in die entsprechende Öffnung eingesetzt wird, die Steuerschaltung entweder vollständig oder in einem wesentlichen Ausmaß durch die vorhandene Elektrode oder die vorhandenen Elektroden in der oder jeder Öffnung in dem Gehäuse unterbrochen wird.

4. Medizinische Vorrichtung nach Anspruch 3, **dadurch gekennzeichnet, daß** dann, wenn die Steuerschaltung unterbrochen wird, sie einen Zustand erfährt, der einen Dauerladungsfluß durch die oder jede Elektrode ermöglicht, so daß die medizinische Vorrichtung im Stand-by-Betrieb arbeiten kann.

5. Medizinische Vorrichtung nach Anspruch 4 in Verbindung mit einem Monitor, **dadurch gekennzeichnet, daß** die medizinische Vorrichtung so ausgebildet ist, daß dann, wenn sie sich im Stand-by-Betrieb befindet, der Monitor den Zustand der oder jeder Elektrode als Funktion der sie passierenden Ladung überwacht.

6. Medizinische Vorrichtung nach Anspruch 5, **dadurch gekennzeichnet, daß** der Monitor vorhanden ist, um den elektrischen Schein-Widerstand oder den Widerstand über der Elektrode zu messen.

7. Medizinische Vorrichtung nach einem der vorhergehenden Ansprüche, **dadurch gekennzeichnet, daß** die medizinische Vorrichtung eine Erfassungsschaltung aufweist, die der oder jeder Öffnung in dem Gehäuse zugeordnet ist, und die Erfassungsschaltung das Vorhandensein einer Elektrode oder einer eine Elektrode enthaltenden Tasche feststellt, sobald die Erfassungsschaltung in die Öffnung oder Öffnungen in dem Gehäuse eingesteckt ist.

8. Medizinische Vorrichtung nach Anspruch 7, **gekennzeichnet durch** einen Schalter zum Einschalten des der Steuerschaltung zufließenden Stroms, so daß die medizinische Vorrichtung beim Entfernen der Elektrode / Tasche aktivierbar ist, um den Strom fließen zu lassen.

9. Medizinische Vorrichtung nach Anspruch 8, **dadurch gekennzeichnet, daß** die Erfassungsschaltung eine Lichtquelle und einen Detektor aufweist, wobei letzterer dazu dient, Unterbrechungen der Lichtquelle festzustellen, sobald eine Elektrode / Tasche in das Gehäuse eingesetzt wird, sowie Lichtemission, sobald die Elektrode / Tasche aus dem Gehäuse entfernt wird, wobei der Detektor dann als Schalter arbeitet, um die medizinische Vorrichtung zu aktivieren.

10. Medizinische Vorrichtung nach Anspruch 9, **dadurch gekennzeichnet, daß** sie eine Kombination aus Schaltelementen aufweist, um zu erfassen und die medizinische Vorrichtung zu aktivieren.

11. Medizinische Vorrichtung nach einem der vorhergehenden Ansprüche, **gekennzeichnet durch** einen Selbsttest-Mechanismus, der so ausgebildet ist, daß dann, wenn die Elektrode bzw. die Elektroden aus dem Gehäuse entfernt werden, eine Schaltung in der medizinischen Vorrichtung komplettiert wird, die entweder bewirken kann, daß die medizinische Vorrichtung betriebsbereit gemacht wird oder die den Selbsttest-Mechanismus aktiviert, so daß Überprüfungen durchgeführt werden können, um sicherzustellen, daß die medizinische Vorrichtung richtig arbeitet, und zwar bevor sie an dem Patienten Verwendung findet.

12. Medizinische Vorrichtung nach Anspruch 11, **dadurch gekennzeichnet, daß** der Selbsttest-Mechanismus zur aktiven oder passiven Überwachung der Monitorschaltung in dem medizinischen Gerät als Folge der Entfernung oder des Einsetzens der Elektroden in das Gehäuse dient, und wobei dann eine Änderung des Schaltzustandes einen Prozessor oder Unterprozessor alarmiert, so daß die Steuerschaltung aktiviert werden kann.

13. Medizinische Vorrichtung nach einem der vorhergehenden Ansprüche, **dadurch gekennzeichnet, daß** sie eine Herz-Lungen-Wiederbelebungsvorrichtung ist.

## Revendications

1. Dispositif médical ayant un circuit de commande apte à actionner une ou plusieurs électrodes en réponse à une analyse de l'état d'un patient par le dispositif médical, le dispositif médical comprenant un support pour une électrode, **caractérisé par le fait que** le dispositif est configuré de telle sorte que, lorsque l'électrode est associée au support, le circuit de commande est désactivé mais lors du retrait de l'électrode à partir du support, le circuit de commande est activé.

2. Dispositif médical selon la revendication 1, ayant un ou plusieurs supports, chacun étant agencé pour être associé à une électrode respective.

3. Dispositif médical selon la revendication 1 ou la revendication 2, dans lequel le ou les supports comprennent des ouvertures dans un boîtier du dispositif médical configurées de telle sorte que, lorsque la ou chaque électrode est introduite dans les ouvertures respectives, le circuit de commande est interrompu soit complètement soit dans une mesure substantielle par la présence de la ou des électrodes dans la ou chaque ouverture dans le boîtier.

4. Dispositif médical selon la revendication 3, dans lequel, lorsque le circuit de commande est interrompu, ledit circuit de commande est agencé pour permettre l'écoulement d'une charge d'entretien à travers la ou chaque électrode, de telle sorte que le dispositif médical peut rester en attente.

5. Dispositif médical selon la revendication 4, comprenant un écran de surveillance, ledit dispositif médical étant configuré de telle sorte que, lorsque le dispositif médical est en veille, l'écran de surveillance est agencé pour surveiller l'état de la ou de chaque électrode en fonction de la charge passant à travers celle-ci.

6. Dispositif médical selon la revendication 5, dans lequel l'écran de surveillance est agencé pour mesurer l'impédance électrique ou la résistance à travers l'électrode.

7. Dispositif médical selon l'une quelconque des revendications précédentes, dans lequel le dispositif médical comprend un circuit de détection associé à la ou à chaque ouverture dans ledit boîtier, le circuit de détection étant disposé pour détecter la présence d'une électrode ou d'une poche contenant une électrode lorsqu'elle est introduite dans la ou les ouvertures dans le boîtier.

8. Dispositif médical selon la revendication 7, comprenant un commutateur pour se commuter en puissance au circuit de commande, de telle sorte que le dispositif médical est activable lors du retrait de l'électrode/de la poche pour permettre l'alimentation.

9. Dispositif médical selon la revendication 8, dans lequel le circuit de détection comprend une source de lumière et un détecteur, le détecteur étant agencé pour détecter une interruption de la source de lumière lorsqu'une électrode/une poche est introduite dans le boîtier et une émission de lumière lorsque l'électrode/la poche est retirée du boîtier, le détecteur agissant ensuite comme commutateur pour activer le dispositif médical.

10. Dispositif médical selon la revendication 9, dans lequel le dispositif médical comprend une combinaison d'éléments de commutation pour détecter et activer ledit dispositif médical.

11. Dispositif médical selon l'une quelconque des revendications précédentes, comprenant un mécanisme d'autotest configuré de telle sorte que, lorsque la ou les électrodes sont retirées du boîtier, un circuit est complété dans le dispositif médical, lequel peut soit amener le dispositif médical à être activé dans un état prêt à être utilisé, soit activer le mécanisme d'auto-test, de telle sorte que des vérifications peuvent être effectuées pour assurer que ledit dispositif médical fonctionne correctement avant d'être utilisé sur le patient.

12. Dispositif médical selon la revendication 11, dans lequel ledit mécanisme d'auto-test est agencé pour surveiller activement ou passivement la commutation dans le dispositif médical à la suite d'un retrait ou d'une introduction d'électrodes dans le boîtier, d'un changement d'état de la commutation alertant ensuite un processeur ou un sous-processeur de telle sorte que le circuit de commande peut être activé.

13. Dispositif médical selon l'une quelconque des revendications précédentes, qui est un dispositif de réanimation cardio-pulmonaire.
